# EUROPEAN PATENT SPECIFICATION

(11) **EP 3 460 043 B1**
(45) Date of publication and mention of the grant of the patent: **29.07.2020**
(21) Application number: 18194708.6
(22) Date of filing: 17.09.2018
(51) Int. Cl.: C12N 1/06

(54) **METHOD FOR LYSING MYCOBACTERIA**
VERFAHREN ZUR LYSE VON MYKOBAKTERIEN
PROCÉDÉ DE LYSE DE MYCOBACTÉRIES

(30) Priority: 20.09.2017 EP 17192039
(43) Date of publication of application: 27.03.2019
(73) Proprietor: Merck Patent GmbH, 64293 Darmstadt (DE)
(72) Inventor: Slaghuis, Joerg, 64297 Darmstadt (DE); Schoenenbruecher, Holger, 63906 Erlenbach am Main (DE); Rossmanith, Peter, 2531 Gaaden (AT); Fuchs, Sabine, 3602 Kirchstetten (AT); Mester, Patrick, 1020 Wien (AT); Wagner, Martin, 1180 Wien (AT)

(56) References cited:
- Takeo Imai: "Preparation of high-molecular- weight DNA: Application to Mycobacterial cells", , 1 January 1994 (1994-01-01), pages 479-482, XP55539282, Retrieved from the Internet: URL:https://ac.els-cdn.com/S00032697847152 0X/1-s2.0-S000326978471520X-main.pdf?_tid= 0cc2cf95-e98e-428c-9db3-08355565ec89&acdna t=1546954755_1cbca5b6a43295c5885484eb32ca8 012 [retrieved on 2019-01-08]
- R PATEL: "Isolation and restriction endonuclease analysis of mycobacterial DNA.", JOURNAL OF GENERAL MICROBIOLOGY, vol. 132, no. 2, 1 February 1986 (1986-02-01), pages 541-551, XP055539197, GB ISSN: 0022-1287, DOI: 10.1099/00221287-132-2-541
- Jt Belisle: "Isolation of mycobacterium species genomic DNA" In: "Mycobacteria protocols; IN: Methods in molecular biology , ISSN 1064-3745 ; Vol. 465", 1 January 2009 (2009-01-01), Humana Press, US, XP055539530, ISBN: 978-1-58829-889-8 vol. 465, pages 1-12, DOI: 10.1007/978-1-59745-207-6_1, * paragraph [1.3.2] *
- A. A. DRIEDGER ET AL: "Rapid lysis of cell walls of Micrococcus radiodurans with lysozyme: effects of butanol pretreatment on DNA", CANADIAN JOURNAL OF MICROBIOLOGY, vol. 16, no. 9, 1 September 1970 (1970-09-01), pages 889-893, XP055538950, CA ISSN: 0008-4166, DOI: 10.1139/m70-151
- MRIDULA BOSE ET AL: "A rapid and gentle method for the isolation of genomic DNA from mycobacteria", NUCLEIC ACIDS RESEARCH, vol. 21, no. 10, 1 January 1993 (1993-01-01), pages 2529-2530, XP055528799, ISSN: 0305-1048, DOI: 10.1093/nar/21.10.2529

## Description

The present invention relates to a method and kit for the lysis of mycobacteria. The sample is treated with a pre-incubation solution that comprises at least an organic solvent and/or ZnCl₂. The resulting pre-incubated sample is then subjected to chemical or enzymatic lysis.

### Background of the invention

Mycobacteria are a large, diverse, and widely distributed family of bacteria that have a high cell-wall lipid content and a slow growth rate. Members of the Mycobacterium genus vary tremendously in virulence. Some are harmless while others like M. tuberculosis are significant pathogens. Many detection methods for determining the presence of pathogenic organisms such as those in the Mycobacteriaceae family rely on the lysis of those organisms and the extraction of their DNA.

Current DNA-Extraction and isolation protocols are not able to efficiently lyse mycobacterial cells. The average extraction efficiencies are presumably -10% and therefore render the possibility of accurate quantification via qPCR almost impossible.

The main reason for the insufficient DNA extraction efficiencies of current protocols is the unique structure of the mycobacterial cell wall which is exceptionally rigid and robust.

Up to today there are basically three different principles to extract DNA and for Mycobacteria mostly combinations of these are used:
1. Thermal: Heating to 99°C over a period of time leads to cell lysis and release of DNA.
2. Physical: Physical disruption of the cells by bead beating, freeze-thaw cycles or sonication (Durnez 2009).
3. Enzymatic/chemical: In a first step the cell wall is enzymatically digested by lysozme and proteinase K. Subsequent to the digestion, the (still intact) cells are chemically lysed with detergents/chaotropes and heat. (Piersimoni 2009, Belisle 2008)

Patel et al. Journal of General microbiology (1986), 132, pages 541-551 suggest a repeated extraction with a mixture of CHCl₃, light petroleum and 150 mM sodium chloride followed by enzymatic lysis.

Imai et al. Analytical Biochemistry (1994), 222, pages 479-482 describe a method for preparing DNA from mycobacterial cells involving the treatment of the cells with an organic solvent (CHCl₃-methanol (2:1, v/v) or ethanol-ether (1:1, v/v)) prior to chemical lysis.

It would be advantageous to have a process for lysing Mycobacteria that is simple, fast, and more effective than the known processes.

### Brief description of the Invention

It has been found that a pre-incubation in an organic solvent and/or a ZnCl₂ solution at elevated temperature makes mycobacteria more accessible to chemical and/or enzymatic lysis. Chemical and enzymatic lysis can be performed according to know methods.

Therefore the present invention relates to a method for lysing mycobacteria by
a) Providing a sample comprising mycobacterial cells
b) Incubating said sample with a solution comprising a C₄ to C₁₀ alkanol or aniline and/or ZnCl₂ at a temperature of 60°C or more, whereby if the ZnCl₂ is present it is present in concentrations between 1 and 100 mM
c) Removing the solution comprising a C4 to C10 alkanol or aniline and/or the ZnCl₂ from the sample
d) Subjecting the sample from step c) to chemical and/or enzymatic lysis. The boiling point of the solution comprising an organic solvent needs to be above the temperature at which the incubation of step b) is performed, i.e. above 60°C.

In a preferred embodiment, the removal in step c) is performed by centrifugation and optionally washing with ethanol and/or an aqueous buffer.

In another preferred embodiment, the organic solvent is a C₄ to C₁₀ aliphatic alkanol or aniline.

In a very preferred embodiment, the organic solvent is butanol, pentanol, hexanol or aniline.

In another preferred embodiment, the incubation in step b) is performed with an organic solvent and/or ZnCl₂ in a concentration between 1 and 100 mM ZnCl₂, preferably between 2.5 and 50 mM ZnCl₂.

In another preferred embodiment, incubation in step b) is performed at a temperature between 60 and 100°C, preferred at a temperature between 80 and 100°C, most preferred at a temperature between 90 and 100°C.

In another preferred embodiment, the incubation in step b) is performed for a time between 30 minutes and 3 hours, most preferred for a time between 1 and 2 hours.

In another embodiment, lysis in step d) is performed by incubation with lysozyme, afterwards digestion with proteinase K and finally chemical lysis with a lysis solution comprising guanidinium hydrochloride and a detergent.

Herein also described is a kit for lysing mycobacteria comprising means for chemical and/or enzymatic lysis as well as an organic solvent and/or a solution of ZnCl₂.

In a preferred embodiment, the organic solvent is butanol, pentanol, hexanol or aniline.

### Figures

**Figure 1****:** Relative DNA-extraction efficiency changes of various pre-incubation buffer systems.
**Figure 2****:** Relative DNA-extraction efficiency changes of incubation time and temperature of butanol, hexanol and aniline pre-incubation buffer systems, compared against a control incubated for 2h at 99°C. See also Example 1.
**Figure 3****:** Summarization of 12 independent experiments regarding the relative DNA-extraction efficiency changes of butanol, hexanol and aniline pre-incubation buffers, calculated against the direct control. See also Example 3.
**Figure 4****:** Relative DNA-extraction efficiency changes of a butanol pre-incubation step before DNA-Extraction with NucleoSpin® or Bead beating, compared to Bead beating standard method. For details see Example 4.
**Figure 5****:** Relative DNA-extraction efficiency changes of ZnCl₂ + Butanol mixtures or splitted incubation protocols. For details see Example 5.
**Figure 6****:** Relative DNA-extraction efficiency changes of ZnCl₂ + hexanol or aniline mixtures or splitted (first / second) incubation protocols.
**Figure 7****:** Relative DNA-extraction efficiency changes of incubation temperature and concentration of ZnCl₂ based pre-incubation buffer systems, compared against a control incubated for 2h at 99°C.
**Figure 8****:** Relative DNA-extraction efficiency changes of a ZnCl₂ pre-incubation step before DNA-Extraction with NucleoSpin® or Bead beating, compared to the Bead beating standard method.

### Description of the invention

It surprisingly turned out that a pre-incubation step makes mycobacteria much more accessible to chemical and/or enzymatic lysis. The pre-incubation does not lyse the mycobacteria. After pre-incubation, the mycobacteria are still intact. It seems that the pre-incubation weakens their cell wall so that it is more accessible to lysis. Pre-incubation means the incubation of the sample prior to lysis by chemical and/or enzymatic methods.

Mycobacteria also called mycobacterial cells to be pre-incubated and lysed according to the present invention are all Mycobacteriaceae.

The term "buffer" as used herein, refers to aqueous solutions or compositions that resist changes in pH when acids or bases are added to the solution or composition. This resistance to pH change is due to the buffering properties of such solutions. Thus, solutions or compositions exhibiting buffering activity are referred to as buffers or buffer solutions. Buffers generally do not have an unlimited ability to maintain the pH of a solution or composition. Rather, they are typically able to maintain the pH within certain ranges, for example between pH 7 and pH 9. Typically, buffers are able to maintain the pH within one log above and below their pKa (see, e.g. C . Mohan, Buffers, A guide for the preparation and use of buffers in biological systems, CALBIOCHEM, 1999). Buffers and buffer solutions are typically made from buffer salts or preferably from non-ionic buffer components like TRIS and HEPES. A stabilized pH value typically contributes to reproducible results. Non- limiting examples of buffers include MES, MOPS, MOPSO, Tris, HEPES, phosphate, acetate, citrate, succinate, and ammonium buffers, as well as combinations of these.

Lysis of cells means to disrupt the cellular wall or to break open cells. Lysis offers the possibility to get access to e.g. DNA or proteins which are within the cells. A person skilled in the art knows thermal, mechanical, enzymatic and chemical ways to lyse cells.

For chemical lysis the cells are typically suspended in a medium comprising a detergent and/or a chaotropic substance. The outer membrane is dissolved and cytosolic components can be extracted. Examples for a chemical lysis are detergent lysis using e.g. SDS, LiDS or sarkosyl in appropriate buffers; the use of chaotropes such as Guanidium hydrochloride (GHCI), Guanidium thiocyanate (GTC), sodium iodide (Nal), perchlorate etc. There are a number of commercially available solutions for this technique.

Enzymatic lysis uses for example lysozyme, proteinases, pronases or cellulases or any of the other lysis enzymes. As mentioned above, all such methods are standard lysis techniques and are well known in the art, and any such method or combination of methods may be used.

For performing the method of the present invention, a sample comprising mycobacteria is treated with the pre-incubation solution according to the present invention at a temperature of 60°C or more. A sample comprising mycobacteria is any sample comprising mycobacterial cells. The sample may also include diluents, buffers, detergents, and contaminating species and the like that are found mixed with the mycobacteria. Examples of suitable samples are clinical samples like blood, sputum or urine, microbiological samples like cell culture samples, as well as tissue samples e.g. of animals.

In one embodiment, the pre-incubation solution is a solution comprising an organic solvent or ZnCl₂. According to the invention organic solvent means a single organic solvent or a mixture of two or more organic solvents.

The organic solvent is preferably a solvent with a boiling point above 100°C. Suitable organic solvents are especially saturated, unsaturated or aromatic hydrocarbons which carry at least one OH and/or NH₂ group, like substituted benzene derivatives, substituted alkanes etc.

In a preferred embodiment, the organic solvent is an aliphatic or aromatic hydrocarbon which is substituted with one OH or one NH₂ group.

In the pre-incubation solution according to the invention, the organic solvent is a C₄ to C₁₀ aliphatic alkanol or aniline.

In an especially preferred embodiment, the organic solvent is n-butanol, n-pentanol, n-hexanol or aniline.

In a preferred embodiment, the pre-incubation solution only contains one or more organic solvents having a boiling point above 60°C, preferably it only contains one organic solvent having a boiling point above 60°C, most preferred the pre-incubation solution is n-butanol, n-pentanol, n-hexanol or aniline.

In another embodiment, the pre-incubation solution comprises an organic solvent and/or ZnCl₂.

If ZnCl₂ is added to an organic solvent it is typically added in form of a highly concentrated aqueous solution. The solution should be more than 1 M, preferably between 2 and 5 M, preferably around 4 M. Preferably ZnCl₂ is used in combination with organic solvents that can be mixed at least slightly with water. For example the ability of butanol to mix with water is sufficient. 4 M ZnCl₂ in water can be mixed with butanol without a separation of the phases or a precipitation of the ZnCl₂. The final concentration of ZnCl₂ in the mixture with the solvent should be between 1 and 100 mM ZnCl₂, preferably between 2.5 and 50 mM.

If no organic solvent is present, the ZnCl₂ is typically added to the sample in an aqueous solution. In such aqueous pre-incubation solutions ZnCl₂ is present in concentrations between 1 and 100 mM ZnCl₂, preferably between 2.5 and 50 mM ZnCl₂.

Preferably the sample is incubated with a pre-incubation solution that comprises either ZnCl₂ or an organic solvent. In one embodiment, the sample is first incubated with a pre-incubation solution comprising an organic solvent and afterwards, after removal of the first pre-incubation solution, with a pre-incubation solution comprising ZnCl₂ or the other way round.

The mixing of the sample with the pre-incubation solution is typically performed by just mixing the sample with the pre-incubation solution. To perform the incubation as effectively as possible, preferably, other solvents are removed from the sample prior to pre-incubation, e.g. by centrifugation. For this, the sample is centrifuged, preferably at 5000 to 10000g for 3 to 10 minutes, the supernatant is removed and only the pellet comprising the mycobacteria is incubated with the pre-incubation solution. The amount of pre-incubation solution is not very critical. It should be enough to cover the sample and suspend the sample in the solution in case of a more solid sample like a pellet. In case of a liquid sample it is preferably at least the same volume as the sample liquid.

Incubation is typically performed for a time between 30 minutes and 3 hours, most preferred for a time between 1 and 2 hours.

The incubation in step b) is performed at a temperature above 60°C, more preferred at a temperature between 60 and 100°C, more preferred at a temperature between 80 and 100°C, most preferred at 90 to 100°C.

Incubation can further be supported by shaking or otherwise actively mixing during incubation.

After incubation, the sample comprising the mycobacteria is typically separated from the incubation solution. This can for example be performed by centrifugation, e.g. 8000 to 16000 rpm for 10 to 30 minutes, and washing. Preferably, the mixture is first centrifuged, the supernatant is removed as fully as possible and the remaining pellet is re-suspended and re-centrifuged one time or several times in a washing solvent like chloroform/methanol and/or ethanol and/or an aqueous buffer. A person skilled in the art is able to perform the washing steps to remove the pre-incubation solution as effectively as possible. In case the incubation solution is an organic solvent typically one or more washing steps with an organic washing solvent are suitable. If the pre-incubation solution is an aqueous solution comprising ZnCl₂, washing with an aqueous washing solvent is typically sufficient.
After the last washing step, the sample can be used for lysis and DNA extraction.

It has been found that the pre-incubation according to the present invention is especially suitable for chemical and/or enzymatic lysis. The pre-incubation according to the present invention results in lysis recovery rates that are up to ten-fold higher, typically around five-fold higher compared to lysis without pre-incubation.

In a preferred embodiment, lysis is performed enzymatically e.g. by incubation with lysozyme and/or proteinase K, followed by chemical lysis. For chemical lysis the cells are typically suspended in a medium comprising a detergent and/or a chaotropic substance. The outer membrane is dissolved and cytosolic components can be extracted. Examples for a chemical lysis are detergent lysis using e.g. SDS, LiDS or sarkosyl in appropriate buffers; the use of chaotropes such as Guanidium hydrochloride (GHCI), Guanidium thiocyanate (GTC), sodium iodide (Nal), perchlorate etc. There are a number of commercially available solutions for this technique.

A suitable lysis procedure is the one performed with the NucleoSpin® tissue kit from Macherey & Nagel, Germany.

It has further been found that the pre-incubation according to the invention cannot be equally effectively combined with mechanical lysis. It seems that the pre-incubation results in a clumping of the cells so that they cannot be effectively lysed by mechanical destruction like bead beating based methods.

After lysis, any further process step like DNA extraction and analysis or protein extraction and analysis can be performed. A person skilled in the art is aware of suitable methods.

Further described herein is a kit for lysing mycobacteria. The kit comprises means for performing the method of the present invention, especially means for chemical and/or enzymatic lysis as well as an organic solvent and/or a solution of ZnCl₂ for performing pre-incubation. Means for chemical and/or enzymatic lysis are detergents like SDS, LiDS and/or sarkosyl in appropriate buffers and/or chaotropes such as Guanidium hydrochloride (GHCI), Guanidium thiocyanate (GTC), sodium iodide (Nal), perchlorate etc.

In a preferred embodiment, the kit comprises reagents for enzymatic and/or chemical lysis as well as n-butanol, n-pentanol, n-hexanol or aniline.

The present invention provides a simple method to improve lysis and DNA extraction of mycobacteria. By pre-incubating the mycobacteria with an organic solvent and/or ZnCl₂ at elevated temperature before lysis the lysis efficiency can be increased remarkably. For mycobacteria most published DNA extraction protocols apply mechanical lysis like bead beating at some point. The method of the present invention provides a method for lysing mycobacteria without applying mechanical lysis methods.

### Examples

The following examples represent practical applications of the invention and are in no way limiting the scope of the invention.

### Materials and Methods

### Pre- incubation for DNA extraction

The Pre-DNA-Extraction procedure was performed as follows: 100µl of a Mycobacterium bovis BCG culture were transferred to a 1.5ml plastic tube (Eppendorf, Hamburg, Germany) centrifuged at 8000 × g for 5min and the supernatant was gently discarded. The bacterial pellet was then re-suspended in 1ml PBS with additional centrifugation at 8000 × g for 5min and the supernatant was discarded to remove possible free DNA from dead cells. The remaining bacterial pellet was then re-suspended with 500µl of the respective pre-DNA-Extraction incubation liquid and thoroughly vortexed for 5s. Afterwards the sample was incubated in a thermocycler at 750rpm for the respective time and temperature. After incubation the sample was centrifuged at 14000 × g for 15min and the supernatant was carefully removed if no clear pellet was visible. To remove all remaining liquid from the incubation buffer, the pellet was washed first with 500µl of 2:1 Chlorform-Methanol and centrifuged at 14000 × g for 15min. Afterwards the remaining pellet was additionally washed with 96% Ethanol and thereafter PBS, with centrifugation at 8000 × g for 5min after each step. After the last washing step the remaining pellet could be used for DNA-Extraction.

### DNA-Extraction

### NucleoSpin® tissue kit

DNA-Extraction from *M. bovis* BCG samples with the NucleoSpin® tissue kit was done according to the manufacturer's instructions and the support protocol for Gram-positive bacteria. The method includes a one hour incubation step with 20mg/ml Lysozyme and an overnight Proteinase K digestion. The final cell disruption is performed by chemical lysis.

### Bead beating based DNA-Extraction (AGES-SOP)

DNA-Extraction from *M. bovis* BCG samples with the DNeasy Blood & Tissue kit was done according to the manufacturer's instructions and the support protocol for mycobacteria form the AGES. The mycobacterial cell pellets were resuspended in 180µl of ATL buffer and transferred to a 2ml Eppendorf tube with stainless steel bead (5mm) from Quiagen. The samples were then homogenized with a MixerMill tissue lyser for 2 x 3min. at 30Hz. After homogenization proteinase K was added and the samples were incubated for 1h at 56°C at 750rpm in an Eppendorf thermomixer. After incubation with proteinase k, the protocol was performed exactly according to the manufacturer's instructions.

### DNA standard and real-time PCR quantification

The genomic DNA of five millilitre culture of *M. bovis* BCG was extracted by using the NucleoSpin® tissue kit (Macherey - Nagel) and the support protocol for Gram-positive bacteria. DNA concentration was analytically determined by an 8452A Diode Array Spectrophotometer (Hewlett Packard, Palo Alto, CA, USA). For the straight calibration line, a six step decimal dilution series of the respective DNA solution, with a concentration of 1 ng/µl, was subjected to qPCR.
qPCR detection of M. bovis BCG was performed by targeting a 141 bp fragment of the *IpqT* gene according to a previously published format (Reddington, 2011). qPCR was performed in an Mx3000p real-time PCR thermocycler (Stratagene, La Jolla, CA, USA). The 25 µl volume contained 5 µl of DNA template. qPCR results were expressed as bacterial cell equivalents (BCE). All qPCR reactions were performed in duplicate.

### Performance testing of pre-incubation buffers systems

The influence of a pre-incubation buffer on the DNA extraction efficiency from *M. bovis* BCG was experimentally determined as following: To remove possible free DNA and damaged cells, 5 ml *M. bovis* BCG cultured cells were centrifuged at 8000 × g for 5min. and the supernatant was discarded. The cells were then re-suspended in 1ml PBS, vigorously vortexed for 5s and centrifuged at 8000 × g for 5min. This washing procedure was repeated twice and the final pellet was resuspended in 2 ml PBS. For the experiments 100 µl of washed cells were transferred to a sterile 1.5 ml Eppendorf cup centrifuged at 8000 × g for 5min., the supernatant was discarded and the pellet was used for further processing. The *M. bovis* BCG cell pellets were then either resuspended in the respective pre-incubation buffers or used directly for DNA extraction with the NucleoSpin® tissue kit. In each experiment every sample condition was tested in technical duplicates and controlled two fold. For the direct control the *M. bovis* BCG cell pellets were used directly for DNA extraction with the NucleoSpin® tissue kit. For the second control the *M. bovis* BCG cell pellets were resuspended in PBS and treated like pre-incubation samples including all incubation and washing steps. After pre-incubation the samples were washed as described in the section and the remaining pellet was used for DNA extraction. The performance of the pre-incubation protocols was calculated against one of the above mentioned controls, depending on the specific experiment, and expressed as relative DNA recovery.

### Experimental data

### 1. Pre-incubation efficiency

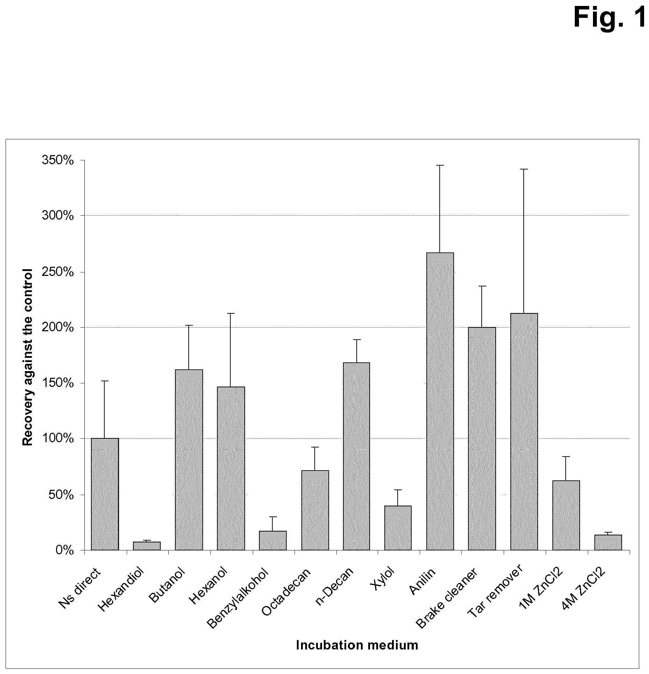

Organic solvents were tested on their effect of improving the DNA extraction efficiency from *M. bovis* BCG cells of a commercial available kit based on enzymatic and chemical cell lysis. The results are shown in Figure 1. From the tested organic solvents aniline enhanced the DNA extraction efficiency the most in comparison to the control. Incubation with butanol, hexanol, n-decan, tar remover (5% non-ionic tensides; >30% aliphatic hydrocarbons; 15-30% aromatic hydrocarbons, solvent) as well as brake cleaner also led to an increase in DNA extraction efficiency. However, n-decan, tar remover and brake cleaner are difficult to handle due to their strong evaporation at elevated temperatures.

### 2. Influence of temperature

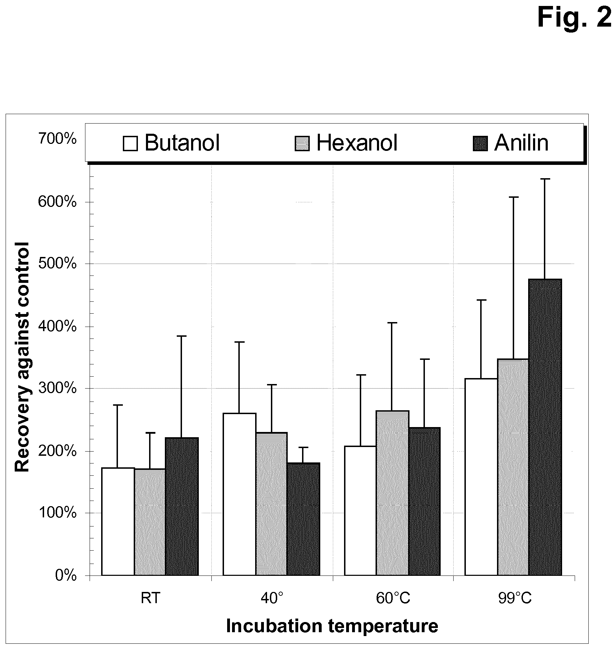

First, the influence of the temperature was examined to optimize the DNA extraction efficiency. In Figure 2 the relationship between different incubation temperatures (RT, 40°C, 60°C and 99°C) for 2h and the DNA extraction efficiency, compared to the control incubated for 2h with PBS, is shown.

The results clearly show that an incubation temperature of 99°C increases the DNA extraction efficiency the most. For all three organic solvents, no efficiency differences between temperatures below 99°C could be observed. If the cells were however incubated at 99°C with the respective solution the DNA extraction efficiency, compared to the lower temperatures, clearly increased.

### 3. Confirmation of pre-incubation efficiency

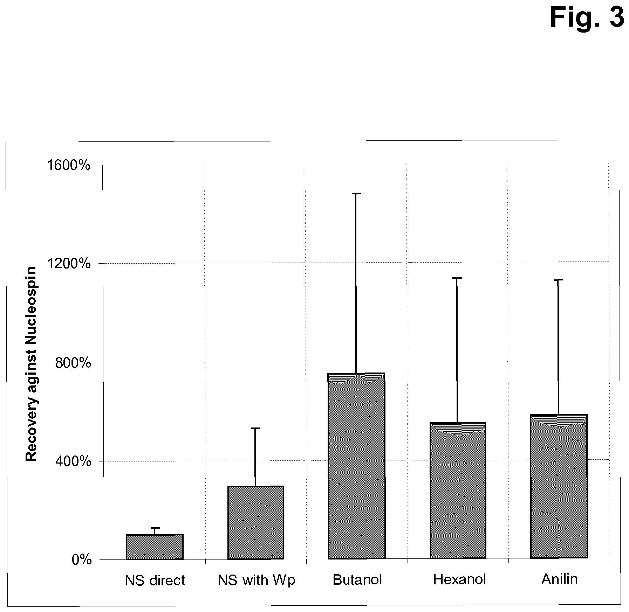

A problematic fact is the high deviation rate between single experiments. One of the major reasons for the high deviation is the clumping of mycobacterial cells when grown in medium, which also hinders other microbiological enumeration techniques. Therefore, the optimized protocol for pre-DNA extraction incubation was repeatedly tested against the respective controls on 12 different days and as technical duplicates. In Figure 3 the results of these experiments are shown for the untreated control (no pre-incubation), the PBS control (2h at 99°C in 1xPBS with subsequent washing steps) and the respective Butanol, Hexanol and Aniline pre-incubation buffers.

The results presented in Figure 3 show that a significant improvement of DNA extraction by applying the developed pre-incubation occurs. Compared to the untreated control, directly subjected into the DNA extraction, all four tested buffer systems had significantly higher recoveries. For the incubation for 2h at 99°C in 1xPBS with subsequent washing protocol a 3fold DNA recovery could be achieved. This is indeed not surprising as a pre-incubation at higher temperatures is known to increase DNA extraction. However, the pre-incubation with the different organic solvents lead to even a 6-8fold average increase in DNA extraction efficiency. The best efficiency increase was obtained by incubation with butanol with an average 7.5 fold recovery compared to the untreated control. Although the pre-incubation with hexanol and Aniline had a lower average recovery than butanol, the results were still significantly higher compared to the untreated but as well the PBS control. These results demonstrate that is not sufficient to only apply heat but that also a chemical pre-treatment according to the present invention is absolutely necessary for correct quantification with qPCR. A reason for the positive effect of the pre-incubation might be the solubilization of the outer lipid layer of Mycobacteria by the organic solvents.

### 4. Combination with mechanical lysis

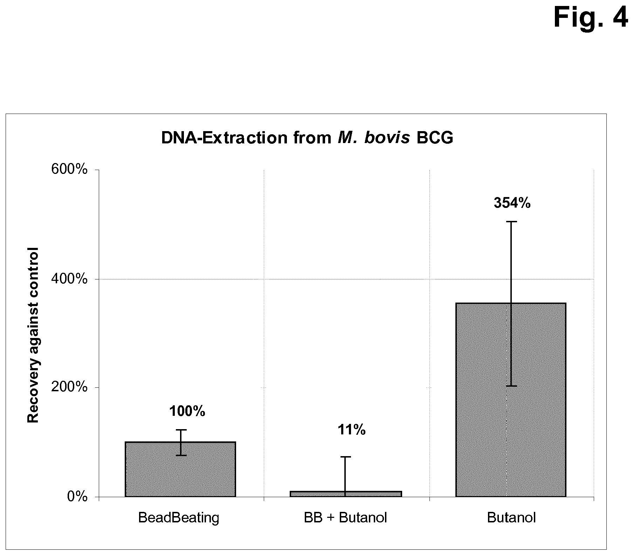

The performance of the pre-incubation protocol of the invention was tested against a previously published DNA extraction protocol that employs Bead beating and is used as a SOP for the Austrian Agency for Health and Food Safety (AGES). The experiments were performed in triplicates and the butanol pre-incubation was used. After pre-incubation with butanol for 2h at 99°C and subsequent washing, DNA extraction from treated *M. bovis* BCG cells was either performed by the Bead beating protocol or with the NucleoSpin® tissue kit as previously performed. The results of all experiments are expressed as recovery compared to the values obtained by the direct Bead beating protocol and are shown in Figure 4.

As the results demonstrate, the pre-incubation with butanol with subsequent DNA extraction via NucleoSpin® resulted in a significantly higher DNA recovery compared to the standard Bead beating protocol, which is considered as gold standard. Interestingly if after the pre-incubation with butanol, the DNA extraction was performed applying Bead beating, the DNA recovery was only 11% compared to the Bead-beating protocol without pre-incubation. This result confirms that the pre-incubation indeed does not lead to cell lysis itself. It makes the cells more sensitive to enzymatic degradation of the cell wall and chemical lysis, as performed with the NucleoSpin® kit. A physical lysis however, as employed by the Bead beating, is indeed hampered by a pre-incubation. The presumed reason for this is an increased clumping and conglomeration of mycobacterial cells.

In conclusion, the results presented above demonstrate the effectiveness of a pre-incubation step with organic solvents at elevated temperature before an enzymatic and/or chemical DNA extraction and purification protocol. The DNA extraction efficiency of the method of the invention resulted in an average of 800% increase compared to an only enzymatic and chemical DNA extraction protocol but also a 300% increase compared to a Bead beating with subsequent enzymatic digestion protocol.

### 5. Pre-incubation with organic solvents and ZnCl₂

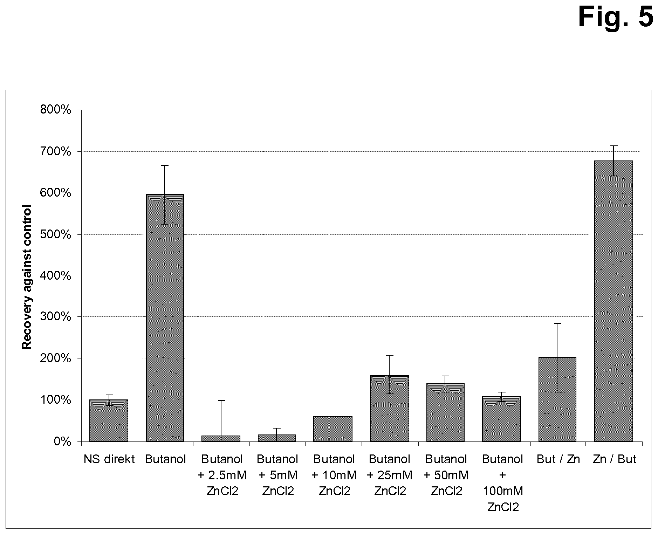

In addition, we found clear evidence that the incubation with small concentrations of ZnCl₂ could greatly improve DNA extraction efficiency. We tried a combination of ZnCl₂ with Butanol to increase its DNA extraction efficiency. In a first set of experiments we tried a co-incubation of ZnCl₂ (2.5-100mM) with Butanol for 2h at 99°C but also a splitted incubation for either 50mM ZnCl₂ with subsequent butanol incubation, each for 1h at 99°C, or vice versa. The results of these experiments are presented in Figure 5.

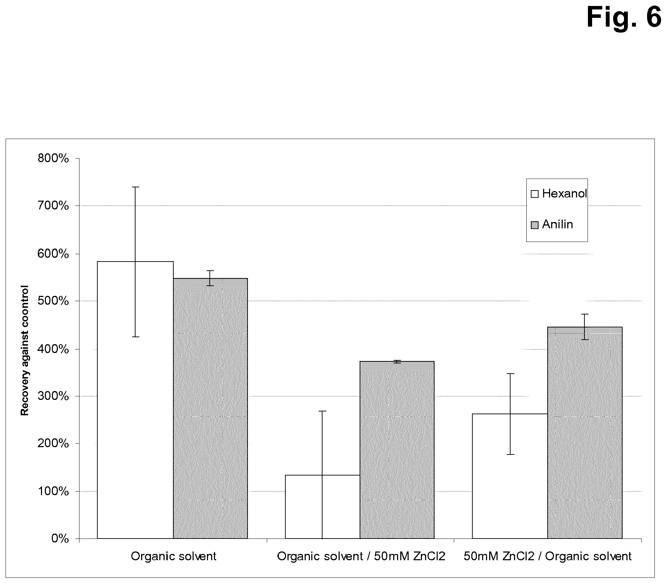

As can be seen in Figure 5, the combination of Butanol and ZnCl₂ between 2.5mM and 10mM resulted in a lower recovery compared to the control. At ZnCl₂ concentration between 25 and 100mM however, the recoveries were slightly better compared to the control (110-180%) but still a lot less than with the optimized Butanol pre-incubation protocol. The samples which were first incubated with butanol and afterwards with ZnCl₂ also showed a lower recovery compared to the only butanol protocol. However, if the samples were incubated first with ZnCl₂ and then with Butanol, the recovery matched those results obtained by the pure butanol protocol. To determine if this effect was only present with butanol, the same experiments were repeated with hexanol and aniline and the results are shown in Figure 6.

In principle, the results obtained with hexanol and aniline were comparable to those of butanol. Again, a combination of the organic solvent and ZnCl₂ resulted in much lower recoveries compared to the pure organic solvent (Data not shown). Concerning the separated incubation experiments the higher recoveries were obtained if the organic solvent was applied after the ZnCl₂ incubation.

### 6. Pre-incubation with ZnCl₂

Experiments with ZnCl₂ in PBS without any organic solvents were performed with 2.5, 25 and 100mM at 4 different temperatures.

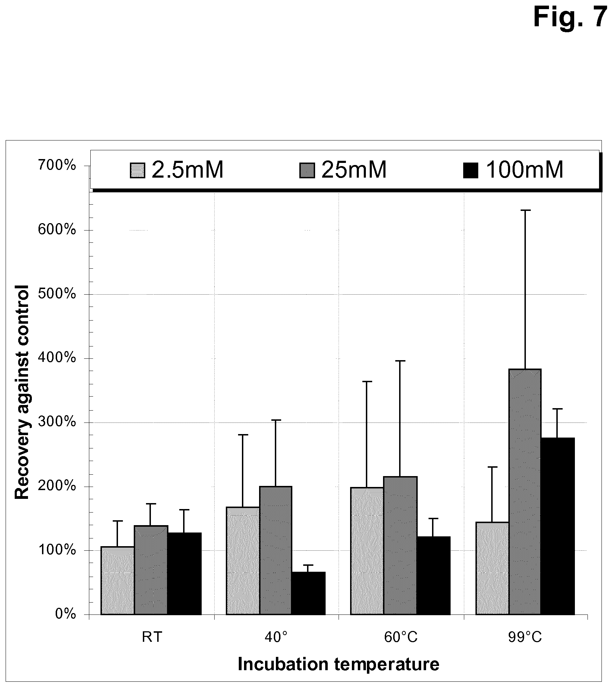

As can be seen in Figure 7 an increase of the incubation temperature leads to a more efficient DNA extraction for all tested concentrations. The best recoveries were obtained with a buffer of 25mM ZnCl₂ and 2h incubation at 99°C. Higher concentrations of ZnCl₂ seem to not positively enhance the DNA extraction efficiency.

### 7. Pre-incubation with ZnCl₂ in combination with mechanical lysis

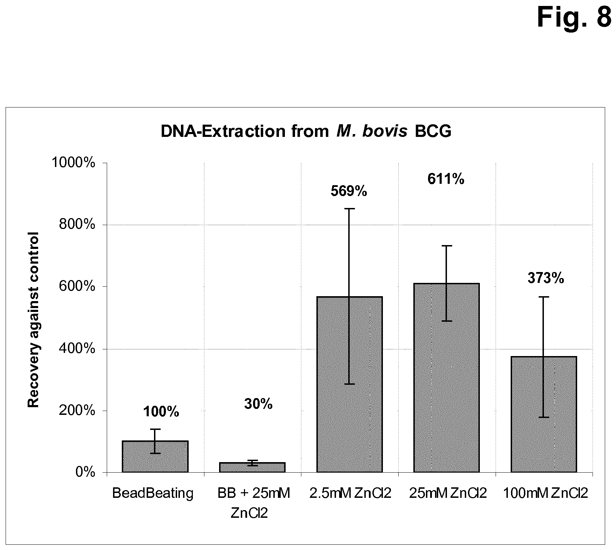

The pre-incubation buffers with ZnCl₂ (2.5, 25 and 100mM) were evaluated against the gold standard Bead beating method. Also it was tested if a pre-incubation with 25mM ZnCl₂ could increase DNA extraction efficiency of the Bead beating method and the results are presented in Figure 8.

The results of these experiments are in good agreement with the previous experiments. In general, it can be concluded that the pre-incubation with ZnCl₂ with subsequent NucleoSpin® results in much higher DNA extraction efficiencies compared to the Bead beating protocol. Also it was confirmed that with higher concentrations (100mM) of ZnCl₂ the DNA extraction efficiency was getting lower compared to the other ZnCl₂ based pre-incubation buffers, although the efficiency was still better in comparison to the Bead beating control. Interestingly the same effect as with organic solvents was observed, namely that a pre-incubation with ZnCl₂ before Bead beating actually resulted in lower DNA extraction efficiency (30% compared to the control). The reason for this effect is presumably the same clumping of *M. bovis* BCG cells as was observed for butanol.

## Claims

1. A method for lysing mycobacteria by
a) Providing a sample comprising mycobacterial cells
b) Incubating said sample with a solution comprising a C₄ to C₁₀ alkanol or aniline and/or ZnCl₂ at a temperature of 60°C or more, whereby if the ZnCl₂ is present it is present in concentrations between 1 and 100 mM
c) Removing the solution comprising a C₄ to C₁₀ alkanol or aniline and/or ZnCl₂ from the sample
d) Subjecting the sample from step c) to chemical and/or enzymatic lysis.

2. Method according to claim 1, **characterized in that** the removal in step c) is performed by centrifugation and optionally washing with ethanol and/or an aqueous buffer.

3. Method according to claims 1 or 2, **characterized in that** in step b) ZnCl₂ is present in concentrations between 2.5 and 50 mM.

4. Method according to one or more of claims 1 to 3, **characterized in that** the incubation in step b) is performed at a temperature between 80 and 100°C.

5. Method according to one or more of claims 1 to 4, **characterized in that** the incubation in step b) is performed for a time between 30 minutes and 3 hours.

6. Method according to one or more of claims 1 to 5, **characterized in that** lysis in step d) is performed by incubation with lysozyme, afterwards digestion with proteinase K and finally chemical lysis.

## Patentansprüche

1. Verfahren zum Lysieren von Mykobakterien durch
a) Bereitstellen einer Probe, die Mykobakterienzellen umfasst,
b) Inkubieren der Probe mit einer Lösung, die ein C₄- bis C₁₀-Alkanol oder Anilin und/oder ZnCl₂ umfasst, bei einer Temperatur von 60°C oder mehr, wobei das ZnCl₂ bei Vorhandensein in Konzentrationen zwischen 1 und 100 mM vorliegt,
c) Entfernen der Lösung, die ein C₄- bis C₁₀-Alkanol oder Anilin und/oder ZnCl₂ umfasst, aus der Probe,
d) Unterziehen der Probe aus Schritt c) einer chemischen und/oder enzymatischen Lyse.

2. Verfahren nach Anspruch 1, **dadurch gekennzeichnet, dass** die Entfernung in Schritt c) durch Zentrifugation und gegebenenfalls Waschen mit Ethanol und/oder einem wässrigen Puffer durchgeführt wird.

3. Verfahren nach den Ansprüchen 1 oder 2, **dadurch gekennzeichnet, dass** in Schritt b) ZnCl₂ in Konzentrationen zwischen 2,5 und 50 mM vorliegt.

4. Verfahren nach einem oder mehreren der Ansprüche 1 bis 3, **dadurch gekennzeichnet, dass** die Inkubation in Schritt b) bei einer Temperatur zwischen 80 und 100°C durchgeführt wird.

5. Verfahren nach einem oder mehreren der Ansprüche 1 bis 4, **dadurch gekennzeichnet, dass** die Inkubation in Schritt b) für eine Zeit zwischen 30 Minuten und 3 Stunden durchgeführt wird.

6. Verfahren nach einem oder mehreren der Ansprüche 1 bis 5, **dadurch gekennzeichnet, dass** die Lyse in Schritt d) durch Inkubation mit Lysozym, anschließende Spaltung mit Proteinase K und schließlich chemische Lyse durchgeführt wird.

## Revendications

1. Méthode destinée à la lyse de mycobactéries, par
a) la mise à disposition d'un échantillon comprenant des cellules mycobactériennes ;
b) l'incubation dudit échantillon avec une solution comprenant un alcanol en C₄ à C₁₀ ou de l'aniline et/ou du ZnCl₂ à une température de 60°C ou plus, où, si le ZnCl₂ est présent, il est présent selon des concentrations comprises entre 1 et 100 mM ;
c) le retrait de la solution comprenant un alcanol en C₄ à C₁₀ ou de l'aniline et/ou du ZnCl₂ à partir de l'échantillon ;
d) la soumission de l'échantillon de l'étape c) à une lyse chimique et/ou enzymatique.

2. Méthode selon la revendication 1, **caractérisée en ce que** le retrait dans l'étape c) est mis en œuvre par centrifugation et éventuellement lavage par de l'éthanol et/ou un tampon aqueux.

3. Méthode selon les revendications 1 ou 2, **caractérisée en ce que**, dans l'étape b), le ZnCl₂ est présent selon des concentrations comprises entre 2,5 et 50 mM.

4. Méthode selon l'une ou plusieurs parmi les revendications 1 à 3, **caractérisée en ce que** l'incubation dans l'étape b) est mise en œuvre à une température comprise entre 80 et 100°C.

5. Méthode selon l'une ou plusieurs parmi les revendications 1 à 4, **caractérisée en ce que** l'incubation dans l'étape b) est mise en œuvre pendant une durée comprise entre 30 minutes et 3 heures.

6. Méthode selon l'une ou plusieurs parmi les revendications 1 à 5, **caractérisée en ce que** la lyse dans l'étape d) est mise en œuvre par incubation avec du lysozyme, puis digestion par de la protéinase K et finalement une lyse chimique.
